# EUROPÄISCHE PATENTSCHRIFT

(11) **EP 2 768 551 B1**
(45) Veröffentlichungstag und Bekanntmachung des Hinweises auf die Patenterteilung: **21.06.2017**
(21) Anmeldenummer: 12808265.8
(22) Anmeldetag: 16.10.2012
(51) Int. Cl.: A61M 5/00, A61M 5/315

(54) **SPRITZE FÜR EINEN HOCHDRUCKINJEKTOR**
SYRINGE FOR A HIGH PRESSURE INJECTOR
SYRINGUE POUR INJECTEUR DE HAUTE PRESSION

(30) Priorität: 19.10.2011 DE 102011054605
(43) Veröffentlichungstag der Anmeldung: 27.08.2014
(73) Patentinhaber: Medtron AG, 66128 Saarbrücken (DE)
(72) Erfinder: ALTMEYER, Hanno, 66538 Neunkirchen (DE); MEHNER, Gotthilf, 66280 Sulzbach (DE)
(74) Vertreter: Wagner Albiger & Partner Patentanwälte mbB
(86) Internationale Anmeldenummer: PCT/EP2012/070514
(87) Internationale Veröffentlichungsnummer: WO 2013/057119

(56) Entgegenhaltungen:
- DE-A1- 10 006 560
- US-A- 5 902 276
- US-A1- 2011 034 882

## Beschreibung

Die Erfindung betrifft eine Spritze für einen Hochdruckinjektor, umfassend einen Spritzenzylinder mit einem an einem patientenseitigen Ende ausgebildeten Schlauchanschluss für eine Schlauchleitung und einen innerhalb des Spritzenzylinders in einer Vorschubrichtung geführten Kolben, der mit Dichtflächen zur abdichtenden Anlage an einer Innenwandung des Spritzenzylinders ausgebildet ist.

Mittels derartiger Spritzen wird eine einem Patienten zu verabreichende Flüssigkeit, z.B. ein Kontrastmittel vorgehalten und in einen Injektor eingebracht, der sodann diese Flüssigkeit gesteuert, z.B. während eines bildgebenden Untersuchungsverfahrens in den Patienten injiziert. Ein solches bildgebendes Untersuchungsverfahren stellt z.B. die Angiografie dar, weiche Hochdruckinjektoren erfordert, die die bereitgestellte Flüssigkeit, wie Kontrastmittel mit einem Druck von bis zu 1.200 psi oder 83 bar (8,3 MPa) aus der hierfür vorgesehenen Spritze in den Patienten injiziert.

Die bisher eingesetzten Spritzen für Hochdruckinjektoren verfügen über einen im Spritzenzylinder geführten Kolben, der aus einem harten und entsprechend formstabilen Kunststoff gefertigt ist, auf dem eine weiche, flexible Kunststoffkappe mit mindestens zwei abgeformten erhabenen Dichtungsringen montiert ist, oder in welchen mindestens zwei Dichtringe eingesetzt werden, um den hohen Arbeitsdrücken von bis zu 83 bar im Rahmen einer Hochdruckinjektion leckagefrei standzuhalten.

Aus der DE 100 06 560 A1 ist ein Kolbenstopfen aus 2 Komponenten bekannt, von denen eine innere Komponente härter ausgebildet und mit einer aus weicherem Material gebildeten Überzugslage versehen ist, die stoffschlüssig mit der inneren Komponente verbunden ist und die Abdichtung im Spritzenzylinder bewirkt.

Neben der guten Abdichtung ist auch der Fehlbedienungssicherheit derartiger Spritzen große Aufmerksamkeit zu widmen, um insbesondere zu verhindern, dass einem Patienten infolge einer solchen Fehlbedienung ein Luftvolumen injiziert wird, welches zu lebensbedrohlichem Komplikationen führen kann.

Aufgabe der vorliegenden Erfindung ist es, eine Spritze für einen Hochdruckinjektor der eingangs genannten Art vorzuschlagen, die sich rationell fertigen lässt, eine zuverlässige Abdichtung auch bei den erreichbaren hohen Injektionsdrücken gewährleistet und druckabhängig die Reibung zwischen Kolben und Spritzenzylinder moduliert und somit die Leistungsaufnahme des Injektors druckabhängig bestimmt und hohe Sicherheit gegen fehlbedienungsbedingte Luftinjektionen bietet.

Zur Lösung der gestellten Aufgabe wird erfindungsgemäß die Ausgestaltung einer Spritze gemäß den Merkmalen des Patentanspruches 1 vorgeschlagen.

Vorteilhafte Ausgestaltungen und Weiterbildungen der Erfindung sind Gegenstand der abhängigen Ansprüche.

Die Erfindung schlägt vor, den Kolben der erfindungsgemäßen Spritze als Zweikomponenten-Spritzgussteil auf Basis thermoplastischer Kunststoffe mit einem Kernteil aus einem härteren Kunststoff und einer auf das Kernteil aufgebrachten Überzugslage aus einem demgegenüber weicheren Kunststoff auszubilden, die thermisch fest mit dem härteren Kunststoff des Kernteiles verbunden ist, so dass der Kolben nach den bekannten Zweikomponenten-Spritzgießverfahren einfach und rationell herstellbar ist und sich zum Zusammenwirken mit konventionellen Spritzenzylindern eignet. Die Überzugslage bildet zumindest die Dichtflächen des Kolbens und die dem patientenseitigen Ende des Spritzenzylinders zugewandte Kolbenoberfläche des Kolbens aus. Zur Aufnahme eventuell in der Spritze enthaltener Luftvolumina weist der Kolben im Bereich seiner Kolbenoberfläche Ausnehmungen auf.

Eine solche Spritze für einen Hochdruckinjektor mit einem als Zweikomponentenspritzgießteil mit härterem Kernteil und demgegenüber weicheren Überzugslage ausgebildeten Kolben lässt sich einfach und preiswert herstellen, bietet hohe Funktionssicherheit und lässt sich z.B. bei einer Sterilisation mit Ethylenoxid vom Sterilisationsmedium besser benetzen und kann somit in einer kürzeren Kontaktzeit sterilisiert werden.

Im Rahmen des bestimmungsgemäßen Gebrauchs einer erfindungsgemäßen Spritze wird an den Schlauchanschluss derselben zunächst eine Schlauchleitung definierter Länge und mit definiertem Querschnitt angeschlossen, über die nachfolgend die Spritze mit der zu injizierenden Flüssigkeit, z.B. einem Kontrastmittel durch Aufziehen derselben befüllt wird. Insoweit gelangt beim Aufziehen der Spritze zunächst das Luftvolumen aus der Schlauchleitung in das Innere des Spritzenzylinders, bevor die aufgezogene Flüssigkeit nachfolgt. Aus diesem Grund wird eine solchermaßen aufgezogene Spritze anschließend entlüftet, indem der Kolben in Vorschubrichtung soweit vorgeschoben wird, dass das enthaltene Luftvolumen ausgepresst wird, bevor die Schlauchleitung mit dem Patienten verbunden und die Injektion durchgeführt wird. Wenn aber infolge einer Fehlbedienung dieses Entlüften der Spritze unterbleibt, besteht die Gefahr der Injektion des Luftvolumens in den Patienten.

Bei der erfindungsgemäßen Ausgestaltung wird diese Gefahr durch die in der Kolbenoberfläche vorhandenen Ausnehmungen gebannt, da sie auch bei vollständig in Vorschubrichtung vorgeschobenem Kolben einen Restraum innerhalb des Spritzenzylinders definieren, in welchem das vorhandene Luftvolumen verbleibt und insoweit nicht in den Patienten injiziert werden kann. Dabei nutzt die Erfindung den Umstand aus, dass sich die Spritzen innerhalb eines Hochdruckinjektors üblicherweise in einer nach unten z.B. um 120 Grad geneigten Lage des Schlauchanschlusses befinden, so dass innerhalb des Spritzenzylinders enthaltene Luft zum Kolben aufsteigt.

Da üblicherweise mit derartigen Spritzen nur speziell zugehörige Schlauchleitungen verwendet werden, ist deren maximales Innenvolumen bekannt und es wird vorgeschlagen, die Ausnehmungen im Kolben so zu gestalten, dass deren Gesamtvolumen dem Innenvolumen der Schlauchleitung entspricht oder dieses übersteigt.

Nach einem Vorschlag der Erfindung weisen die Dichtflächen mindestens eine Dichtlippe auf, die an der Innenwandung des Spritzenzylinders anlegbar ist.

Ein weiterer Vorschlag der Erfindung sieht vor, dass zwei Dichtlippen vorgesehen sind, von denen eine Dichtlippe in Vorschubrichtung und die andere Dichtlippe in entgegengesetzter Richtung vorsteht. Hierdurch wird eine noch höhere Dichtigkeit im angestrebten Druckbereich der Spritze von bis zu 83 bar bei gleichzeitig verbesserten Laufeigenschaften des Kolbens erzielt. Die in Vorschubrichtung vorstehende Dichtlippe ist somit der Hydraulikseite und die in entgegengesetzter Richtung vorstehende Dichtlippe der Pneumatikseite des Kolbens zugewandt.

Weiterhin wird vorgeschlagen, eine Dichtlippe in Vorschubrichtung vorstehend ausgebildet und durch einen in Vorschubrichtung offenen Freiraum vom übrigen Korpus des Kolbens beabstandet ist, während eine weitere Dichtlippe entgegen der Vorschubrichtung vorstehend ausgebildet und durch einen entgegen der Vorschubrichtung offenen Freiraum vom übrigen Korpus des Kolbens beabstandet ist.

Durch diese Konfiguration der Dichtlippen wird gewährleistet, dass diese mit steigendem Injektionsdruck des Injektors eine entsprechend steigende Flächenpressung auf die Innenwandung des Spritzenzylinders ausüben und somit zuverlässigen Schutz gegen Leckage auch bei zu erwartenden hohen Drücken im Rahmen des Einsatzes an einem Hochdruckinjektor bietet.

Nach einem Vorschlag der Erfindung sind die in Vorschubrichtung weisende Kolbenoberfläche und eine kegelige Spitze des Kolbens von der Überzugslage gebildet, so dass eine besonders einfache Herstellung gewährleistet ist. Bei dieser Ausgestaltung bedeckt somit die Überzugslage sämtliche mit der im Spritzenzylinder einfüllbaren Flüssigkeit in Kontakt kommenden Oberflächen des Kolbens bzw. des Kernteiles desselben.

Sofern zwei Dichtlippen zum Einsatz kommen, sind diese nach einem Vorschlag der Erfindung entgegen der Vorschubrichtung durch eine Nut voneinander beabstandet, wodurch ein besonders gleichmäßiges Gleiten des Kolbens innerhalb des Spritzenzylinders ohne die Gefahr einer Verkantung und der damit einhergehenden Schwächung der Dichtfunktion der Dichtlippe gewährleistet ist. Sofern unerwarteterweise Flüssigkeit eine Dichtlippe überwinden sollte, wird diese innerhalb der in diesem Fall als Auffangreservoir dienenden Nut aufgefangen und an einem Austreten zwischen Kolben und Spritzenzylinder gehindert.

Das Kernteil des Kolbens kann nach einem Vorschlag der Erfindung aus einem geeignet harten Polycarbonat oder einem ggf. verstärkten Acryl-Butadien-Styrol hergestellt sein.

Die Überzugslage wird nach einer Ausgestaltung der Erfindung aus einem mit dem Kernteil haftfest verbindbaren thermoplastischen Elastomer mit einer Shore-Härte von 60 - 90 A gebildet. Beispiele für ein mit dem Kernteil aus Polycarbonat oder Acryl-Butadien-Styrol haftfest verbindbaren thermoplastischen Elastomer für die Überzugslage umfassen beispielsweise thermoplastische Elastomere auf Basis von PP/EPDM. Beispielsweise können die unter den Handelsbezeichnungen Sarlink® der Teknor Apex Company oder Badaprene® der Bada AG vertriebenen teilvernetzten EPDM/PP-Blends für die Ausbildung der Überzugslage Verwendung finden. Auch können Polyurethane entsprechender Härte als Alternative in Betracht kommen.

Das Kernteil kann darüber hinaus mit einem angeformten Kupplungszapfen für die Verbindung mit der Kolbenstange eines Injektors ausgebildet sein. Schließlich kann das Kernteil an seinem entgegen der Verschieberichtung liegenden Ende mit einer Bodenplatte versehen sein, die z.B. aus Polycarbonat oder Acryl-Butadien-Styrol hergestellt sein kann. Die Bodenplatte dient zur axialen Stabilisierung des Kernteiles und auch als Druckaufnehmer für die Kolbenstange des Injektors.

Weitere Ausgestaltungen und Einzelheiten der Erfindung werden nachfolgend anhand der Ausführungsbeispiele darstellenden Zeichnungen näher erläutert. Es zeigen:
- Figur 1: einen Längsschnitt durch eine erfindungsgemäße Spritze;
- Figur 2: den Schnitt D-D durch die Spritze gemäß Figur 1;
- Figur 3a: in schematischer Darstellung den Schnitt durch eine erste Ausführungsform eines Kolbens der erfindungsgemäßen Spritze gemäß der Linie A-A in Fig. 3b;
- Figur 3b: die Aufsicht auf den Kolben gemäß Fig. 3a
- Figur 4a: in schematischer Darstellung den Halbschnitt durch eine zweite Ausführungsform eines Kolbens der erfindungsgemäßen Spritze gemäß der Linie B-B in Fig. 4b;
- Figur 4b: die Aufsicht auf den Kolben gemäß Fig. 4a

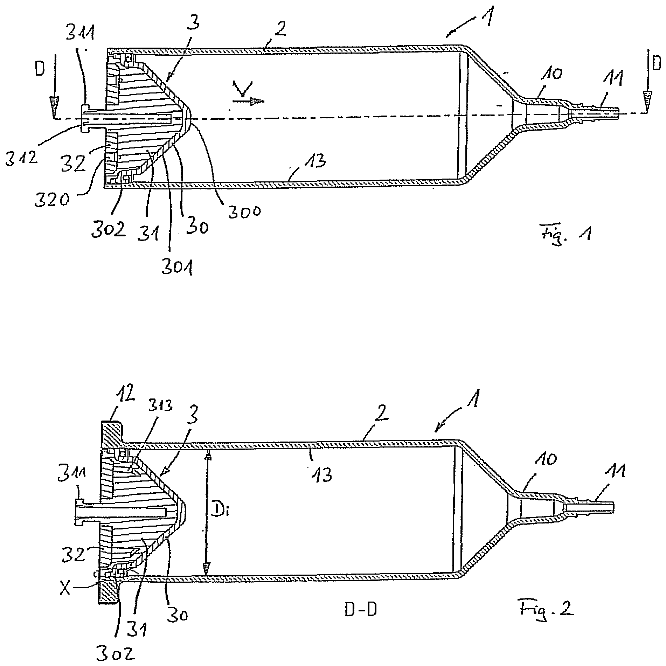

Aus den Figuren 1 und 2 ist eine Spritze 1 für einen Hochdruckinjektor ersichtlich, die als Zubehörteil für einen Hochdruckinjektor Verwendung findet und dazu dient, eine in den Patienten zu verabreichende Flüssigkeit, z.B. Kontrastmittel für ein bildgebendes Untersuchungsverfahren bereitzustellen.

In an sich bekannter Weise umfasst die Spritze 1 einen Spritzenzylinder 2, der beispielsweise aus einem PETG oder Polycarbonat hergestellt sein kann oder auch aus POM mit in der Matrix eingearbeitetem Gleitmittel gefertigt sein kann. Dieser Spritzenzylinder 2 ist üblicherweise transparent.

Innerhalb des Spritzenzylinders 2 ist ein Kolben 3 in einer Verschieberichtung V zum Zwecke des Injizierens der Flüssigkeit aus dem Spritzenzylinder 2 verschieblich geführt, wobei der Kolben 3 selbstverständlich auch in entgegengesetzter Richtung zum Aufziehen der Spritze bewegbar ist. Der größte Durchmesser des Kolbens 3 korrespondiert insoweit mit dem Innendurchmesser Di des Spritzenzylinders 2.

Der Spritzenzylinder 2 umfasst an seinem patientenseitigen Ende 10 einen Schlauchanschluss 11 für eine hier nicht dargestellte Schlauchleitung zum Patienten, während der Kolben 3 einen Kupplungszapfen 311 zum Verbinden mit der hier nicht dargestellten Kolbenstange des Injektors aufweist. Der Befestigung am Injektor dienen überdies Flanschvorsprünge 12 am rückwärtigen Ende des Spritzenkolbens 2, siehe Figur 2.

Der Kolben 3 ist als Zweikomponenten-Spritzgussteil auf Basis thermoplastischer Kunststoffe ausgebildet und umfasst ein Kernteil 31 aus einem härteren Kunststoff, z.B. Polycarbonat oder Acryl-Butadien-Styrol (ABS), welches ggf. verstärkt sein kann. Auf dieses Kernmaterial 31 ist in der Zweikomponenten-Spritzgusstechnik haftfest ein geeignetes weicheres Kunststoffmaterial, beispielsweise ein thermoplastisches Elastomer oder ein Polyurethan mit in der Matrix eingearbeitetem Gleitmittel und mit einer Shore-Härte von 60 - 90 A als Überzugslage 30 aufgebracht, die zugleich sämtliche mit der im Inneren des Spritzenkolbens 2 vorhaltbaren Flüssigkeit in Kontakt kommenden Oberflächen des Kolbens 3 bedeckt, die nachfolgend erläutert werden.

So weist der Kolben eine Spitze 300 mit umgebender Kolbenoberfläche 301 auf, die in einer Dichtfläche 302 endet, weiche abdichtend an der Innenwandung 13 des Spritzenkolbens 2 anliegt. Die Spitze 300, die Kolbenoberfläche 301 und die hieran anschließende Dichtfläche 302 sind aus der weicheren Überzugslage 30 integral gebildet.

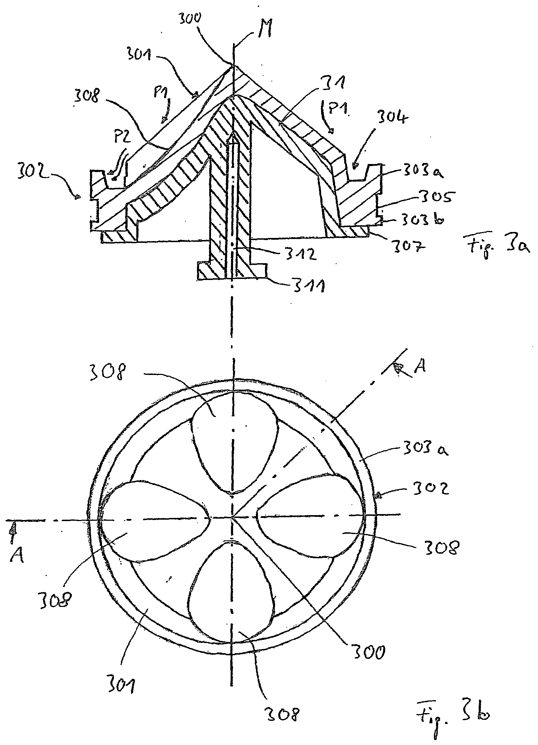

Der Aufbau der Dichtfläche 302 ist in näheren Einzelheiten auch aus der schematisierten Darstellung gemäß Figur 3a ersichtlich.

Ausgehend von der Kolbenoberfläche 301 umfasst die Dichtfläche 302 eine in Vorschubrichtung V vorstehende Dichtlippe 303 a, die durch einen in Vorschubrichtung V geöffneten nutartigen Freiraum 304 von der übrigen Kolbenoberfläche 301 separiert ist. An die Dichtlippe 303 a schließt sich entgegen der Vorschubrichtung V betrachtet zunächst eine ringförmige Nut 305 und anschließend eine weitere Dichtlippe 303 b sowie ein Absatz 307 an. Im gewählten Ausführungsbeispiel weisen die Dichtlippen 303 a und 303 b bezogen auf die Mittelachse M gleichen Durchmesser auf, der mit dem Innendurchmesser Di des Spritzenzylinders 2 korrespondiert, während die ringförmige Nut 305 und der Absatz 307 ebenfalls gleiche und gegenüber den Dichtlippen 303 a, b, verringerte Durchmesser in Bezug auf die Mittelachse M aufweisen.

Bei bestimmungsgemäßem Vorschub des Kolbens 3 innerhalb des Spritzenzylinders 2 wirken bei den hohen Arbeitsdrücken eines hierzu verwendeten Hochdruckinjektors von bis zu 83 bar entsprechende Gegenkräfte der über das patientenseitige Ende 10 ausgetriebenen Flüssigkeit auf den Kolben 3 und dessen Dichtlippe 303 a ein, was mit Pfeilen P1 in der schematisierten Darstellung gemäß Figur 3a ersichtlich ist. Aufgrund der kegelig zu einer Spitze 300 ansteigenden Form der Kolbenoberfläche 301 tritt somit Flüssigkeit in den in Vorschubrichtung V geöffneten Freiraum 304 ein, siehe Pfeile P2 und bewirkt eine zum Injektionsdruck proportionale Erhöhung der Flächenpressung der Dichtlippe 303 a auf die Innenwandung 13 des Spritzenzylinders 2 im Bereich F, so dass eine zuverlässige Abdichtung in diesem Bereich auch bei höchsten Drücken gewährleistet ist. Sollte dennoch Flüssigkeit die Dichtlippe 303 a überwinden, wird diese in der dahinter befindlichen ringförmigen Nut 305 gesammelt und von der Dichtlippe 303 b an einem Austreten aus der ringförmigen Nut 305 gehindert. Die Dichtlippe 303 b bewirkt außerdem eine exakt axiale Führung des Kolbens entlang der Innenwandung 13 des Spritzenzylinders 2.

Das Aufziehen, d.h. Befüllen einer derartigen Spritze mit der zu injizierenden Flüssigkeit erfolgt im Allgemeinen durch eine an den Schlauchanschluss 11 angeschlossene Schlauchleitung, die auch später die Verbindung zum Patienten herstellt. Insofern gelangt beim Aufziehen der Spritze zunächst die in der Schlauchleitung enthaltene Luft in das Innere des Spritzenzylinders 2, bevor die Flüssigkeit nachfolgt und diese Luft muss anschließend durch einen begrenzten Vorschub des Kolbens 3 in Vorschubrichtung entfernt werden, um eine Luftinjektion in den Patienten zu verhindern. Wird dieses Entlüften vergessen, kann eine sehr bedrohliche Situation für den Patienten eintreten.

Um dieser Gefahr zu begegnen, ist die Kolbenoberfläche 301 des Kolbens mit mehreren, hier vier um je 90 Grad zueinander versetzt angeordneten Ausnehmungen 308 in der Kolbenoberfläche 301 ausgebildet, deren Volumen gegenüber der gedachten Rotationsfläche des Kolbens zumindest dem Volumen des Lumens der Schlauchleitung entspricht. Bei einem Vorschub des Kolbens (3) in Vorschubrichtung (V) bis zu dessen vorderster Stellung kann von daher ein z.B. durch nicht erfolgte Entlüftung im Spritzenzylinder (2) vorhandenes Luftvolumen vollständig innerhalb dieser Ausnehmungen 308 aufgenommen werden und wird zuverlässig an einem Austrag aus der Spritze in den Patienten gehindert. Damit erreicht die dargestellte Spritze höchsten Schutz gegen Fehlbedienung.

Man erkennt ferner, dass der Kolben 3 im Bereich seines Kernteiles 31 mit Hohlräumen 313 für eine verbesserte Lastaufnahme und für eine verringerte Zykluszeit bei der Herstellung des Kolbens 3 ausgebildet ist, ferner ist vom rückwärtigen Ende her der Kupplungszapfen 311 an das Kernteil 31 angeformt und weist eine Bohrung 312 auf.

Zur Verbesserung der Lastaufnahme der am Kupplungszapfen 311 angreifenden und hier nicht dargestellten Kolbenstange ist überdies eine Bodenplatte aus Polycarbonat oder ABS in das Kernteil 31 eingesetzt und z.B. mittels Schrauben lagegesichert.

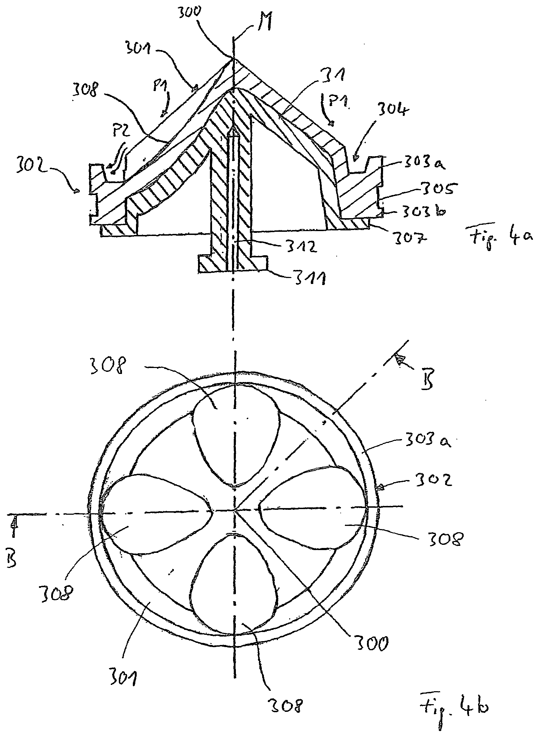

Die Figuren 4a und 4b zeigen eine gegenüber dem vorangehend dargestellten Ausführungsbeispiel abgewandelte Form des Kolbens 3, bei dem gleiche Teile gleiche Bezugsziffern aufweisen und zur Vermeidung von Wiederholungen nicht nochmals gesondert erläutert werden, sofern dies nicht zum Verständnis der Erfindung erforderlich ist.

Im Unterschied zum Ausführungsbeispiel gemäß Figuren 3a und 3b umfasst der Kolben 3 eine zweite Dichtlippe 303 b, die entgegen der Vorschubrichtung V vorstehend ausgebildet und ebenfalls über einen Freiraum 304 b vom übrigen Korpus des Kolbens 3 abgesetzt ist, wie es auch bei der in Vorschubrichtung V abstehenden Dichtlippe 303 a und deren Freiraum 304 a der Fall ist. Hierdurch ergeben sich bessere Eigenschaften des Kolbens 3 bei der Druckbeaufschlagung und dessen Laufeigenschaften. Die erste Dichtlippe 303 a ist demgemäß der Hydraulikseite und die zweite Dichtlippe 303 b der Pneumatikseite des Kolbens zugeordnet. Die erste Dichtlippe 303 a wirkt vornehmlich bei Bewegung des Kolbens in Vorschubrichtung V, während bei entgegengesetzter Bewegung des Kolbens primär die zweite Dichtung 303 b wirkt.

Es versteht sich, dass die den Zeichnungen entnehmbaren Abmessungen und geometrischen Gestaltungen des Spritzenzylinders 2 und des Kolbens 3 lediglich beispielhaft sind und sich diese nach Maßgabe des verwendeten Injektors und des Anwendungsgebietes richten.

In jedem Fall ist es erfindungsgemäß möglich, Spritzen herzustellen, die auch für Anwendungen in Hochdruckinjektoren bei Injektionsdrücken von bis zu 1.200 psi, entsprechend 83 bar betriebssicher und dicht sind.

## Patentansprüche

1. Spritze mit einer angeschlossenen Schlauchleitung eines mit Injektionsdrücken bis zu 83 bar (8,3 MPa) injizierenden Hochdruckinjektors, die Spritze umfassend einen Spritzenzylinder (2) mit einem an einem patientenseitigen Ende (10) ausgebildeten Schlauchanschluss (11) für die Schlauchleitung und einen innerhalb des Spritzenzylinders (2) in einer Vorschubrichtung (V) geführten Kolben (3), der mit Dichtflächen (302) zur abdichtenden Anlage an einer Innenwandung (13) des Spritzenzylinders (2) ausgebildet ist und als Zweikomponenten-Spritzgussteil auf Basis thermoplastischer Kunststoffe mit einem Kernteil (31) aus einem härteren Kunststoff und einer auf das Kernteil (31) aufgebrachten Überzugslage (30) aus einem demgegenüber weicheren Kunststoff ausgebildet ist, wobei die Überzugslage (30) zumindest die Dichtflächen (302) und die dem patientenseitigen Ende (10) des Spritzenzylinders zugewandte Kolbenoberfläche (301) des Kolbens (3) ausbildet, **dadurch gekennzeichnet, dass** der Kolben (3) eine Spitze (300) mit umgebender Kolbenoberfläche (301) aufweist und der Kolben (3) im Bereich seiner Kolbenoberfläche (301) mit Ausnehmungen (308) in der Kolbenoberfläche (301) ausgebildet ist, deren Volumen gegenüber der gedachten Rotationsfläche des Kolbens mindestens dem Volumen der an den Schlauchanschluss (11) angeschlossenen Schlauchleitung entspricht und die Dichtflächen (302) mindestens eine in Vorschubrichtung (V) vorstehende und durch einen in Vorschubrichtung V geöffneten nutartigen Freiraum von der Kolbenoberfläche (301) separierte Dichtlippe (303a) umfassen, die an der Innenwandung (13) des Spritzenzylinders (2) anlegbar ist, wobei bei Vorschub des Kolbens mittels des Hochdruckinjektors in Vorschubrichtung (V) Flüssigkeit in den Freiraum (304) eintritt und eine zum Injektionsdruck proportionale Erhöhung der Flächenpressung der Dichtlippe (303 a) auf die Innenwandung (13) des Spritzenzylinders (2) bewirkt.

2. Spritze nach Anspruch 1, **dadurch gekennzeichnet, dass** zwei Dichtlippen (303 a, 303 b) vorgesehen sind, die durch eine Nut (305) voneinander beabstandet sind.

3. Spritze nach einem der Ansprüche 1 oder 2, **dadurch gekennzeichnet, dass** eine Dichtlippe (303 a) in Vorschubrichtung (V) vorstehend ausgebildet und durch einen in Vorschubrichtung (V) offenen Freiraum (304 a) vom übrigen Korpus des Kolbens (3) beabstandet ist, während eine weitere Dichtlippe (303 b) entgegen der Vorschubrichtung (V) vorstehend ausgebildet und durch einen entgegen der Vorschubrichtung (V) offenen Freiraum (304 b) vom übrigen Korpus des Kolbens (3) beabstandet ist.

4. Spritze nach einem der Ansprüche 1 bis 3, **dadurch gekennzeichnet, dass** das Kernteil (31) des Kolbens (3) aus Polycarbonat oder Acryl-Butadien-Styrol hergestellt ist.

5. Spritze nach einem der Ansprüche 1 bis 4, **dadurch gekennzeichnet, dass** die Überzugslage (30) aus einem mit dem Kernteil (31) haftfest verbindbaren thermoplastischen Elastomer mit einer Shore-Härte von 60 - 90 A gebildet ist.

6. Spritze nach einem der Ansprüche 1 bis 5, **dadurch gekennzeichnet, dass** das Kernteil (31) mit einem angeformten Kupplungszapfen (311) für eine Kolbenstange eines Injektors ausgebildet ist.

7. Spritze nach einem der Ansprüche 1 bis 6, **dadurch gekennzeichnet, dass** das Kernteil (31) an seinem entgegen der Vorschubrichtung (V) liegenden Ende mit einer Bodenplatte (32) versehen ist.

## Claims

1. Syringe having a connected hose line of a high-pressure injector which can inject at injection pressures up to 83 bar (8.3 MPa), the syringe comprising a syringe cylinder (2) having for the hose line a hose connection (11) which is formed at a patient-side end (10) and a piston (3) which is guided inside the syringe cylinder (2) in an advance direction (V) and which is constructed with sealing faces (302) for sealing abutment against an inner wall (13) of the syringe cylinder (2) and which is constructed as a two-component injection-moulded component on the basis of thermoplastic plastics materials with a core component (31) of a harder plastics material and a covering layer (30) which comprises a softer plastics material and which is applied to the core component (31), wherein the covering layer (30) at least forms the sealing faces (302) and the piston surface (301) of the piston (3) facing the patient-side end (10) of the syringe cylinder, **characterised in that** the piston (3) has a tip (300) with a surrounding piston surface (301) and the piston (3) in the region of the piston surface (301) thereof is constructed with recesses (308) in the piston surface (301) whose volume with respect to the notional surface of revolution of the piston corresponds at least to the volume of the hose line which is connected to the hose connection (11) and the sealing faces (302) comprise at least one sealing lip (303a) which protrudes in the advance direction (V) and which is separated from the piston surface (301) by means of a groove-like free space which is open in the advance direction V and which can be placed on the inner wall (13) of the syringe cylinder (2), wherein, when the piston is advanced by means of the high-pressure injector in the advance direction (V), fluid enters the free space (304) and brings about an increase of the surface pressure of the sealing lip (303a) on the inner wall (13) of the syringe cylinder (2), which increase is proportional to the injection pressure.

2. Syringe according to claim 1, **characterised in that** there are provided two sealing lips (303a, 303b) which are spaced apart from each other by means of a groove (305).

3. Syringe according to either claim 1 or 2, **characterised in that** a sealing lip (303a) is constructed so as to protrude in the advance direction (V) and is spaced apart from the remaining body of the piston (3) by means of a free space (304a) which is open in the advance direction (V), whilst another sealing lip (303b) is constructed so as to protrude counter to the advance direction (V) and is spaced apart from the remaining body of the piston (3) by means of a free space (304b) which is open counter to the advance direction (V).

4. Syringe according to any one of claims 1 to 3, **characterised in that** the core component (31) of the piston (3) is produced from polycarbonate or acrylonitrile butadiene styrene.

5. Syringe according to any one of claims 1 to 4, **characterised in that** the covering layer (30) is formed from a thermoplastic elastomer material which can be connected to the core component (31) in an adhesive manner and which has a Shore hardness of from 60 - 90 A.

6. Syringe according to any one of claims 1 to 5, **characterised in that** the core component (31) is constructed with a formed-on coupling journal (311) for a piston rod of an injector.

7. Syringe according to any one of claims 1 to 6, **characterised in that** the core component (31) is provided with a base plate (32) at the end thereof located counter to the advance direction (V).

## Revendications

1. Seringue avec une tuyauterie souple raccordée d'un injecteur à haute pression injectant des pressions d'injection allant jusqu'à 83 bar (8,3 MPa), laquelle seringue comprend un cylindre de seringue (2) avec un raccord de tuyau (11) conçu au niveau de l'extrémité côté patient (10) pour la tuyauterie souple et un piston (3) qui est guidé dans un sens d'avance (V) à l'intérieur du cylindre de seringue (2) et qui est conçu avec des surfaces d'étanchéité (302) destinées à un appui étanchéifiant contre une paroi intérieure (13) du cylindre de seringue (2) et est conçu comme une pièce moulée par injection à deux composants à base de matières synthétiques thermoplastiques avec une partie centrale (31) en une matière plus dure et avec une couche de revêtement (30) appliquée sur la partie centrale (31) en une matière comparativement plus molle, la couche de revêtement (30) formant au moins les surfaces d'étanchéité (302) et la surface de piston (301) du piston (3) qui est tournée du côté de l'extrémité côté patient (10) du cylindre de seringue, **caractérisée en ce que** le piston (3) comporte une pointe (300) avec une surface de piston enveloppante (301), **en ce que** le piston (3) est conçu dans la zone de sa surface de piston (301) avec des évidements (308) dans la surface de piston (301) qui ont un volume qui, par rapport à la surface de rotation imaginaire du piston, correspond au moins au volume de la tuyauterie souple raccordée au raccord de tuyau (11) et **en ce que** les surfaces d'étanchéité (302) comprennent au moins une lèvre d'étanchéité (303a) qui dépasse dans le sens d'avance (V) et est séparée de la surface de piston (301) par un espace libre du type rainure ouverte dans les sens d'avance V et qui peut être placée contre la paroi intérieure (13) du cylindre de seringue (2), de sorte que, lors de l'avance du piston dans le sens d'avance (V) au moyen de l'injecteur à haute pression, du liquide entre dans l'espace libre (304) et provoque une augmentation, proportionnelle à la pression d'injection, de la pression superficielle de la lèvre d'étanchéité (303a) sur la paroi intérieure (13) du cylindre de seringue (2).

2. Seringue selon la revendication 1, **caractérisée en ce qu'**il est prévu deux lèvres d'étanchéité (303a, 303b) qui sont distantes l'une de l'autre grâce à une rainure (305).

3. Seringue selon l'une des revendications 1 ou 2, **caractérisée en ce qu'**une lèvre d'étanchéité (303a) est conçue de manière à dépasser dans le sens d'avance (V) et est distante du reste du corps du piston (3) grâce à un espace libre (304a) ouvert dans le sens d'avance (V) tandis qu'une autre lèvre d'étanchéité (303b) est conçue de manière à dépasser dans le sens contraire du sens d'avance (V) et est distante du reste du corps du piston (3) grâce à un espace libre (304b) ouvert dans le sens contraire du sens d'avance (V).

4. Seringue selon l'une des revendications 1 à 3, **caractérisée en ce que** la partie centrale (31) du piston (3) est fabriquée en polycarbonate ou en acryl-butadiène styrol.

5. Seringue selon l'une des revendications 1 à 4, **caractérisée en ce que** la couche de revêtement (30) est formée à partir d'un élastomère thermoplastique, d'une dureté Shore de 60 - 90 A, pouvant être assemblé à la partie centrale (31) avec une bonne résistance d'adhérence.

6. Seringue selon l'une des revendications 1 à 5, **caractérisée en ce que** la partie centrale (31) est conçue avec un pivot d'accouplement formé (311) pour une tige de piston d'un injecteur.

7. Seringue selon l'une des revendications 1 à 6, **caractérisée en ce que la** partie centrale (31) est munie d'une plaque de fond (32) au niveau de son extrémité située à l'opposé du sens d'avance (V).
